# EUROPÄISCHE PATENTSCHRIFT

(11) **EP 0 765 182 B1**
(45) Veröffentlichungstag und Bekanntmachung des Hinweises auf die Patenterteilung: **09.09.1998**
(21) Anmeldenummer: 95922505.3
(22) Anmeldetag: 06.06.1995
(51) Int. Cl.: B01D 3/38, C11D 3/386, C12P 1/00, C12N 9/52

(54) **VERFAHREN ZUR DESODORIERUNG UND STABILISIERUNG BIOTECHNOLOGISCH GEWONNENER WERTSTOFFE UND IHRER WÄSSRIGEN ZUBEREITUNGEN**
PROCESS FOR DEODORISING AND STABILISING BIOTECHNOLOGICALLY EXTRACTED VALUABLE SUBSTANCES AND THEIR AQUEOUS PREPARATIONS
PROCEDE DE DESODORISATION ET DE STABILISATION DE SUBSTANCES D'INTERET OBTENUES PAR DES BIOTECHNOLOGIES ET DE LEURS PREPARATIONS AQUEUSES

(30) Priorität: 15.06.1994 DE 4420730
(43) Veröffentlichungstag der Anmeldung: 02.04.1997
(73) Patentinhaber: Henkel Kommanditgesellschaft auf Aktien, 40191 Düsseldorf (DE)
(72) Erfinder: RÄHSE, Wilfried, D-40589 Düsseldorf (DE); PAATZ, Kathleen, D-40589 Düsseldorf (DE); PICHLER, Werner, A-6250 Kundl (AT); UPADEK, Horst, D-40883 Ratingen (DE)
(86) Internationale Anmeldenummer: EP9502142
(87) Internationale Veröffentlichungsnummer: WO9534358

(56) Entgegenhaltungen:
- EP-A- 0 554 818
- DE-A- 4 307 115
- GB-A- 1 063 393

## Beschreibung

Die biotechnologische Herstellung von Wertstoffen und Wertstoffgemischen durch Züchtung ausgewählter Mikroorganismen-Populationen hat heute eine beträchtliche großtechnische Bedeutung. Lediglich beispielhaft seien hier die Gebiete der Herstellung von wasch- und/oder reinigungsaktiven Enzymen, insbesondere aus den Klassen der Proteasen, Cellulasen, Lipasen und Amylasen, sowie die Gewinnung pharmakologisch aktiver Wertstoffe benannt. Als primäre Verfahrensprodukte fallen wäßrige Zubereitungen von intracellulären und/oder extracellulären Wertstoffen an, die durch eine Vielzahl von Begleitstoffen - beispielsweise Anteile des Nährmediums - verunreinigt sind. Es besteht ein umfangreicher druckschriftlicher Stand der Technik zur Aufarbeitung solcher Kulturbrühen, wobei hier lediglich auszugsweise benannt seien die Veröffentlichung W. Rähse et al. "Mikrofiltration von Fermenterbrühen", Chem.-Ing.-Tech. 57 (1985), Nr. 9, 747 bis 753 und die darin zitierte Primärliteratur. Aus dem Bereich der Patentliteratur sei wiederum lediglich beispielhaft verwiesen auf die EP-B1 0 200 032; WO 93/16173 und die älteren deutschen Patentanmeldungen DE 43 10 506 und DE 43 22 229.

Die durch eine Mehrzahl von Arbeitsstufen gekennzeichnete Aufarbeitung solcher biotechnologisch gewonnener wäßriger Kulturlösungen sieht insbesondere die möglichst weitgehende Abtrennung unerwünschter Bestandteile durch eine gegebenenfalls mehrstufige Filtration und/oder durch Waschverfahren sowie in der Regel eine nachfolgende Verfestigung der wäßrigen Wertstoffzubereitung unter Mitverwendung von festen Trägersubstanzen vor. Auf dem großtechnisch besonders bedeutungsvollen Gebiet der bakteriologischen Gewinnung von wasch- und reinigungsaktiven enzymatischen Wirkstoffen liegt eine besondere Problematik im nachfolgenden Sachverhalt: Die Biomasse-haltigen Fermenterbrühen enthalten aufgrund des Stoffwechsels im Fermentationsprozeß geringe Mengen an niedermolekularen Verbindungen, insbesondere entsprechenden Stickstoffverbindungen, die sich durch einen extrem aufdringlichen unangenehmen Eigengeruch auszeichnen und den Abbau der biotechnologischen Wertstoffe unter Bildung weiterer unangenehm riechender Verbindungen fördern können. Ein typisches Beispiel für diese Erscheinungen findet sich bei der biotechnologischen Gewinnung von Proteasen bzw. Proteaselösungen, die für den Einsatz in Wasch- und Reinigungsmitteln, insbesondere als Mischungskomponente in Textilwaschmitteln, Verwendung finden sollen. Zu der bereits genannten Literatur des Standes der Technik wird zusätzlich verwiesen auf die internationale Patentanmeldung WO 91/2792. Analog dem in der deutschen Patentschrift DE 29 25 427 beschriebenen Verfahren wird hier unter Einsatz ausgewählter Mikroorganismen (Bacillus licheniformis - ATCC 53926) eine Biomasse-haltige Fermenterbrühe erhalten, die ca. 70.000 Proteaseeinheiten pro Gramm (PE/g) enthält. Die Fermenterbrühe beinhaltet neben gelösten Proteasen, Salzen, Proteinen und Stoffwechselprodukten auch ungelöste Bestandteile wie Bacilluszellen, Mediumreste und Schleimstoffe. Während der Aufarbeitung der Fermenterbrühen (WO 92/11 347) werden die Proteasen stabilisiert sowie gröbere Partikel und die ungelösten Bestandteile durch Dekantieren bzw. Mikrofiltration abgetrennt. Die Aufkonzentrierung der Proteaselösung erfolgt mittels Ultrafiltration und anschließender Eindampfung im Vakuum. Die jetzt gewonnene Proteaselösung wird mit festen Trägermaterialien und Granulierhilfsmitteln vermischt und zu Enzymgranulaten für den Einsatz in Waschmitteln verarbeitet.

Trotz der aufwendigen Reinigungs- und Aufarbeitungsprozesse enthält die aufkonzentrierte Proteaselösung noch immer geringe Mengen an niedermolekularen Stickstoffverbindungen mit unangenehmem Eigengeruch, die zusätzlich den Proteaseabbau bei der Lagerung unter Bildung weiterer unangenehm riechender Schwefelverbindungen - zum Beispiel Merkaptane oder Thioether - beschleunigen können.

Die britische Patentanmeldung GB-A-1 063 393 beschreibt zwar das Entfernen von Substanzen, welche einen unangenehmen Eigengeruch aufweisen, mittels Dampf; jedoch wird an keiner Stelle erwähnt, daß für diesen Zweck überhitzter Dampf eingesetzt werden kann. Vielmehr wäre der Fachmann davon ausgegangen, daß durch hohe Temperaturen das biotechnologische Material beschädigt werden könnte, so daß durch diese Schrift der Einsatz von überhitztem Wasserdampf nicht nahegelegt wird.

Die erfindungsgemäße Lehre geht von der Aufgabe aus in einem zusätzlichen einfach durchzuführenden Arbeitsschritt diese insbesondere niedermolekularen Geruchsstoffe wenigstens so weitgehend zu entfernen, daß - insbesondere nach der Praxis-üblichen Einkapselung der Wertstoffe bzw. Wertstoffgemische - praktisch geruchsneutrale Produkte vorliegen, gleichzeitig aber auch selbstinitiierte Zersetzungsprozesse ausgeschlossen oder zumindest weitgehend unterdrückt werden. Die erfindungsgemäße Lehre wird dabei im nachfolgenden anhand der Gewinnung entsprechend aufbereiteter wasch- und reinigungsaktiver Proteasen aus entsprechenden biotechnologisch gewonnenen Fermenterbrühen geschildert. Die erfindungsgemäße Lehre schränkt sich aber nicht auf dieses spezielle Beispiel ein. Die im Rahmen der Erfindungsbeschreibung geschilderten Prinzipien können in breitem Umfange auf dem hier angesprochenen Gebiet der Wertstoffe und Wertstofflösungen aus der biotechnologischen Herstellung sowie auf verwandten Gebieten eingesetzt werden.

### Gegenstand der Erfindung

Gegenstand der Erfindung ist in einer ersten Ausführungsform ein Verfahren zur Abreicherung von Geruchsstoffen aus fließfähigen wäßrigen Wertstoffzubereitungen aus der Aufarbeitung biotechnologisch gewonnener Kulturbrühen, daß dadurch gekennzeichnet ist, daß man die fließfähige Zubereitung als versprühtes Gut mit einem Dampfstrom behandelt, der - bezogen auf die Arbeitsbedingungen der Sprühzone - als überhitzter Dampf vorliegt.

Das erfindungsgemäße Verfahren eignet sich insbesondere für die Desodorierung und Stabilisierung wäßriger Zubereitungen aus der Aufarbeitung biotechnologischer Kulturbrühen, insbesondere entsprechender Fermenterbrühen, wobei die Behandlung der versprühten Flüssigphase mit dem überhitzten Wasserdampfstrom in der Sprühzone bevorzugt unter verringertem Druck durchgeführt wird. Durch die Wahl dieses Verfahrensparameters des verringerten Druckes in der Sprühzone wird in der im nachfolgenden im einzelnen noch geschilderten Weise die sichere Regelung der Maximaltemperatur des tropfenförmig versprühten Gutes in der Sprühzone im Kontakt mit dem überhitzten Wasserdampf möglich.

In einer weiteren Ausführungsform betrifft die Erfindung die Anwendung des Verfahrens zur Desodorierung von fließfähigen wäßrigen Wertstoffzubereitungen durch deren Behandlung in der Sprühzone mit überhitztem Wasserdampf zur Langzeitstabilisierung der Wertstoffaktivitäten und zur Abreicherung der Geruchsstoffe aus wäßrigen Einsatzmaterialien, die gelöste und/oder dispergierte und auf biotechnologischem Weg gewonnene Wertstoffe enthalten.

### Einzelheiten zur erfindungsgemäßen Lehre

In der älteren nicht vorveröffentlichten internationalen Patentanmeldung WO-A-94/20187 wird ein verbessertes Verfahren zur destillativen Trennung von Mehrstoffgemischen durch Dämpfen beschrieben. Gegenstand der Lehre dieses älteren Schutzrechtes ist in einer ersten Ausführungsform das Verfahren zur Intensivierung und/oder Beschleunigung der destillativen Trennung von Mehrstoffgemischen wenigstens anteilig organischen Ursprungs durch Dämpfen mit bei Arbeitsdruck überhitztem Wasserdampf zum erleichterten Austrag wasserdampfflüchtiger Anteile des Einsatzgutes, wobei dieses Verfahren dadurch gekennzeichnet ist, daß man ein unter Arbeitsbedingungen fließfähiges Einsatzgut in feinteilig versprühter Form dämpft, dabei dessen Versprühen mit Hilfe eines Treibgases vornimmt und hierbei überhitzten Wasserdampf als Treibgas einsetzt.

In einer Abwandlung dieses Verfahrens betrifft die genannte ältere Schutzrechtsanmeldung die dadurch gekennzeichnete Ausgestaltung, daß ein wenigstens weitgehend wasserfreies und unter Arbeitsbedingungen flüssiges Einsatzgut ohne Mitverwendung des Treibgases in einen Strom des überhitzten Wasserdampfes versprüht wird. In dieser älteren Patentanmeldung sind zahlreiche Anwendungsgebiete und spezielle Anwendungsbeispiele für diese Technologie der Desodorierung durch Dämpfen benannt, das erfindungsgemäß betroffene Arbeitsgebiet insbesondere wasserhaltiger Wertstoffzubereitungen aus dem Gebiet biotechnologischer Prozesse ist dort allerdings nicht geschildert. Der Offenbarungsgehalt dieser hier genannten älteren Anmeldung sowie die darin beschriebenen Hinweise auf weiteres druckschriftliches Material wird hiermit ausdrücklich auch zum Gegenstand der vorliegenden Erfindungsoffenbarung gemacht.

Die in dieser älteren Anmeldung vorgesehene Arbeitsweise des Versprühens der zu reinigenden bzw. zu desodorierenden fließfähigen Wertstoffzubereitung unter Mitverwendung eines Treibgases und dabei mit überhitztem Wasserdampf als Treibgas kann auch im Rahmen der jetzt erfindungsgemäß betroffenen Ausführungsform zum Einsatz kommen. Bevorzugt wird erfindungsgemäß aber das flüssige Einsatzgut ohne Mitverwendung eines Treibgases in einen Strom des überhitzten Wasserdampfes versprüht. Gegenüber der zuvor geschilderten Variante in der älteren Anmeldung zeichnet sich die hier betroffene erfindungsgemäße Lehre jetzt dadurch aus, daß das zu desodorierende und/oder zu stabilisierende Einsatzgut in der Regel in Form wäßriger Wertstoffzubereitungen vorliegt. Fermenterbrühen und andere auf biotechnologischem Weg gewonnene Primärprodukte fallen in aller Regel als wasserhaltige Zubereitungen mit beträchtlichen Wassergehalten an. So beträgt der Wassergehalt der auch erfindungsgemäß zu reinigenden Einsatzmaterialien im allgemeinen wenigstens 15 Gew.-% und vorzugsweise wenigstens 35 bis 40 Gew.-%. In aller Regel wird gerade auf dem Gebiet der Wasch- und Reinigungsmittelenzyme schon als Produkt primärer und sekundärer Reinigungsstufen eine wäßrige Wertstoffe enthaltende Phase anfallen, deren Wassergehalt bei wenigstens etwa 50 Gew.-% und beispielsweise im Bereich von 60 bis 80 Gew.-% liegt. Extracelluläre Waschmittelproteasen enthaltende und durch gegebenenfalls mehrfache Filtrationsstufen gereinigte sowie durch zusätzliche Vakuumeindampfung aufkonzentrierte wäßrige Lösungen enthalten in der Regel etwa 55 bis 75 Gew.-% wäßrige Phase vor der nachfolgenden Abmischung mit insbesondere festen Trägerstoffen. Solche wasserhaltigen Wertstoffzubereitungen sind das erfindungsgemäß bevorzugte Einsatzmaterial für die Desodorierung und Stabilisierung durch Behandlung in der Sprühzone mit dem überhitztem Wasserdampf. Dabei wird in der bevorzugten Ausführungsform ohne Mitverwendung eines Treibgases gearbeitet. Die wäßrige Proteaselösung wird unter den im nachfolgenden im einzelnen geschilderten Verfahrensbedingungen vorzugsweise im Gegenstrom mit einem die Sprühzone durchströmenden überhitzten Wasserdampfstrom behandelt. Diese Behandlung kann dabei einstufig oder auch mehrstufig erfolgen. In der Regel wird eine mehrstufige Behandlung in der Sprühzone bevorzugt sein. Dabei kann diese mehrstufige Behandlung durch anteilsweise Kreislauf- bzw. Rückführung der wäßrigen Phase in die Sprühzone bewirkt werden, möglich ist aber auch die Hintereinanderschaltung einer Mehrzahl von Sprühzonen. Dabei können solche kaskadenförmig angeordneten Sprühzonen jeweils unter gleichen oder unterschiedlichen Arbeitsbedingungen gefahren werden. Für die hinreichende Desodorierung und Stabilisierung von wäßrigen Proteaselösungen kann eine 2- bis 5-malige Behandlung der wäßrigen Einsatzlösung in der Sprühzone zu befriedigenden Reinigungseffekten führen.

Lebensfähige Organismen bzw. Organismenbestandteile enthaltende Wertstoffe und Wertstoffgemische wie Enzyme oder andere Produkte biotechnologischer Kulturen sind häufig ausgesprochen temperatursensitiv. Insbesondere gilt dies für Waschmittelenzyme, beispielsweise Proteasen der geschilderten Art. Die Aufarbeitung des biologischen Gutes hat sich darauf einzustellen. Selbstverständlich gilt das insbesondere auch für die erfindungsgemäß vorgesehene Behandlungsstufe mit dem überhitzten Wasserdampf. Die erfindungsgemäße Lehre regelt und erfüllt diese Vorgabe in sehr einfacher Weise durch die Vorgabe und Kontrolle der maximalen Guttemperatur in der Sprühzone durch Einstellung und Regulierung der Druckbedingungen in dieser Sprühzone. In der Regel wird erfindungsgemäß die Sprühzone im Vakuum betrieben. Dabei bestimmt das konkret in der Sprühzone eingestellte Vakuum die Siedetemperatur des Wassers. Das mit dem flüssigen Einsatzgut in vergleichsweise großen Mengen zugeführte Wasser übernimmt damit gleichzeitig die Schutzfunktion der Temperaturregulierung in der Sprühzone. Der überhitzte Wasserdampfstrom, der gegebenenfalls mit Temperaturen der Sprühzone zugeführt wird, die beträchtlich oberhalb dieses Siedepunktes liegen, kann zu keiner unerwünschten Aufheizung des Guttropfens in der Sprühzone führen, solange eine hinreichende Konzentration an flüssiger wäßriger Phase im Guttropfen gewährleistet ist. Die mit dem überhitzten Dampf zugeführte thermische Energie wird durch entsprechende Verdampfung eines Wasseranteiles aufgefangen, ohne daß es zu einer substantiellen Temperatursteigerung im Guttropfen kommt. Die erfindungsgemäß einzusetzenden Arbeitsbedingungen machen von dieser Naturgesetzlichkeit Gebrauch. Bevorzugt werden dabei Arbeitsbedingungen in der Sprühzone, bei denen praktisch keine oder nur eine sehr beschränkte gleichzeitige Aufkonzentration der wäßrigen Einsatzphase stattfindet. Zumindest gilt diese Regel für stark temperaturgefährdete Wertstoffe von der Art der Waschmittelproteasen.

Gewünschtenfalls kann aber das Gesamtverfahren auch dahingehend modifiziert werden, daß vergleichsweise stark verdünnte Einsatzlösungen der biotechnologischen Wertstoffe der Sprühzone zugeführt und hier nicht nur desodoriert sondern gleichzeitig auch anteilsweise aufkonzentriert werden.

Im einzelnen kann auf die genannte ältere Anmeldung gemäß WO-A-94/20187 verwiesen werden.

Für die erfindungsgemäße Reinigung und Stabilisierung von Waschmittelproteasen kann es zweckmäßig sein, Guttemperaturen in der Sprühzone bei Werten von höchstens etwa 45 bis 50°C und vorzugsweise im Bereich von höchstens etwa 35 bis 40°C einzuhalten. In besonders bevorzugten Ausführungsformen werden Guttemperaturen in der Sprühzone von höchstens etwa 30°C eingestellt, wobei die Arbeitsbedingungen - und damit insbesondere das in der Sprühzone einzustellende Vakuum - häufig auf maximale Siedetemperaturen des Wassers im Bereich von etwa 20 bis 25°C gewählt werden. Die konkrete Wahl des jeweiligen Arbeitsdruckes in der Sprühzone ergibt sich aus der naturgesetzlichen Abhängigkeit des Siedepunktes des Wassers von dem eingestellten Arbeitsdruck. In der Praxis können Arbeitsdrucke in der Sprühzone im Bereich von etwa 10 bis 250 mbar, vorzugsweise im Bereich von etwa 15 bis 50 mbar zweckmäßig sein.

Der der Sprühzone zur Reinigungsbehandlung zugeführte Wasserdampfstrom kann mit Einsatztemperaturen zur Verwendung kommen, die beträchtlich über der Siedetemperatur des Wasser unter den Arbeitsbedingungen liegen. So kann die Einsatztemperatur des zugeführten Wasserdampfes wenigstens 50°C und vorzugsweise wenigstens 100°C oberhalb der Siedetemperatur des Wassers beim Arbeitsdruck der Reinigungsstufe liegen. Geeignet können Einsatztemperaturen sein, die wenigstens 150 bis 200°C oberhalb der Siedetemperatur des Wassers beim Arbeitsdruck der Reinigungsstufe liegen. Bezogen auf Normaldruck liegen erfindungsgemäß bevorzugte Eintrittstemperaturen für den Arbeitsdampf im Bereich bis 250°C und vorzugsweise bis 200°C, wobei hier Temperaturen im Bereich von etwa 130 bis 200°C besonders bevorzugt sein können.

Das zu reinigende wäßrige Einsatzgut wird in bevorzugten Ausführungsformen der Sprühzone mit einer Eigentemperatur zugeführt, die wenigstens etwa der Siedetemperatur des Wasser unter Arbeitsbedingungen entspricht. Dabei werden aber naturgemäß insbesondere die limitierenden Faktoren zu berücksichtigenden sein, die sich aus der Temperatursensitivität des biologischen Wertstoffes ableiten. In der Regel liegt damit für das praktische Arbeiten - beispielsweise im Rahmen der Reinigung wäßriger Proteaselösungen - der Temperaturbereich für das flüssige, der Sprühzone zuzuführende Einsatzgut im Bereich von etwa 20 bis 35 oder 40°C.

Einer weiteren Besonderheit der erfindungsgemäß zu behandelnden wäßrigen Enzymlösungen kann Bedeutung zukommen: Wäßrige Kulturlösungen biotechnologischer Wertstoffe zeichnen sich häufig durch ein gewisses thixotropes Verhalten aus. Durch geeignete Hilfsmaßnahmen, insbesondere durch Eintrag von Scherkräften in das zu reinigende Gut, kann die Fließfähigkeit der wäßrigen Wertstoffabmischung sichergestellt werden. Insbesondere ist auch bei der Auslegung und Ausgestaltung der Sprühzone darauf zu achten, daß der zuverlässige Austrag des versprühten Gutes sichergestellt ist. Insgesamt kann die Auslegung der Arbeitsbedingungen für die erfindungsgemäße Sprühreinigung in dem Fachmann an sich bekannter Weise durch diesen Effekt mitbestimmt werden. Das nachfolgende Verfahrensbeispiel gibt hierzu charakteristische Arbeitsparameter an.

### Beispiel

Eine Biomasse enthaltende Fermenterbrühe mit einem Gehalt von ca. 70.000 Proteaseeinheiten pro Gramm (PE/g), hergestellt durch Fermentation von Bacillus licheniformis (ATCC 53926) analog dem in der deutschen Patentschrift DE 29 25 427 angegebenen Verfahren, zeichnet sich durch einen unerwünscht starken Eigengeruch aus. Diese Fermenterbrühe beinhaltet neben gelösten Proteasen, Salzen, Proteinen und Stoffwechselprodukten auch ungelöste Bestandteile wie Bacilluszellen, Mediumreste und Schleimstoffe. Die primär anfallende Fermenterbrühe wird zunächst gemäß den Angaben der internationalen Patentanmeldung WO 92/11 347 aufgearbeitet, wobei gröbere Partikel und ungelöste Bestandteile durch Dekantieren bzw. Mikrofiltration abgetrennt werden. Die Aufkonzentrierung der so vorgereinigten Proteaselösung erfolgt mittels Ultrafiltration und anschließender Eindampfung im Vakuum. Die so aufkonzentrierte Proteaselösung kann mit festen Trägermaterialien und Granulierhilfsmitteln vermischt und zu Enzymgranulaten für den Einsatz in Waschmitteln verarbeitet werden.

Auch die so aufgearbeitete Proteaselösung - und damit auch noch die Enzymgranulate - zeichnen sich durch einen unerwünschten Eigengeruch aus. Die hierfür in geringer Menge vorliegenden niedermolekularen Stickstoffverbindungen beschleunigen zudem den Proteaseabbau, der wiederum zur Bildung weiterer unangenehm riechender Schwefelverbindungen (z.B. Merkaptane, Thioether) führt.

Zur Entfernung der Geruchsstoffe aus der wäßrigen Proteaselösung wird diese nach der Vakuumeindampfung in einem zusätzlichen Verfahrensschritt im Sinne der erfindungsgemäßen Lehre in der Sprühzone desodoriert. Im einzelnen gelten dafür die folgenden Verfahrensparameter:

Die wäßrige Enzymlösung wies einen Trockensubstanzgehalt (Enzyme + Fermentationsrückstände) von 35% und folgende Stoffdaten bei 25°C auf:
- Dichte:: 1.100 kg/m³

**Tabelle 1**

| Viskosität der wäßrigen Enzymlösung in Abhängigkeit von Schergefälle | |
|---|---|
| Schergefälle (S⁻¹) | Viskosität (mPas) |
| 32,5 | 160 |
| 54,3 | 116 |
| 106,0 | 81,8 |
| 150,9 | 69,8 |
| 252,3 | 57,0 |
| 420,3 | 50,0 |
| 701,3 | 43,5 |
| 1169,0 | 41,0 |

Während der Desodorierung wurde die Enzymlösung von oben in einen Behälter gesprüht. Die Enzymlösung sammelte sich am Behälterboden und konnte bei Kreislauffahrweise erneut der Düsenvorrichtung zugeführt werden. Der überhitzte Wasserdampf trat, gleichmäßig verteilt, im Gegenstrom zu der Flüssigkeit in den Desodorierungsbehälter ein.

Der Austrag des Dampfes und der mitgerissenen Geruchsstoffe erfolgte über ein Vakuumsystem mit Kondensation des Wasserdampfes. Der Druck im Behälter lag bei 20 mbar absolut. Die Tabelle 2 beinhaltet die Abmessungen des Desodorierungsbehälters, der Düse und die eingestellten Betriebsparameter.

**Tabelle 2**

| Abmessung des Desodorierapparates und Betriebsparameter der Versuche mit Proteaselösung Abmessung | |
|---|---|
| 1. Desodorierbehälter: | |
| Durchmesser | 300 mm |
| Höhe | 1.200 mm |

| 2. Hohlkegeldüse | |
|---|---|
| Durchmesser | 0,6 mm |
| mittlerer volumetrischer Tropfendurchmesser | ca. 60 µm |

| Betriebsparamter | |
|---|---|
| - Proteaselösung | |
| Durchfluß | 10 kg/h |
| Düsenvordruck | 10 bar |
| Temperatur | 25°C |

| - Wasserdampf | |
|---|---|
| Durchfluß | 1,7 kg/h |
| Druck | 1 bar abs. |
| Temperatur | 170°C |

Die Proteaselösung wurde dreimal über die Desodorierungsstufe rezirkuliert und anschließend wie oben beschrieben zu dem fertigen Enzymgranulat verarbeitet. Die Geruchsnoten der Enzymgranulate verbesserten sich von 4,5 - 5 auf 3 (Bewertung nach dem Schulnotensystem 1 - 5).

## Patentansprüche

1. Verfahren zur Abreicherung von Geruchsstoffen aus fließfähigen wäßrigen Wertstoffzubereitungen aus der Aufarbeitung biotechnologisch gewonnener Kulturbrühen, dadurch gekennzeichnet, daß man die fließfähige Zubereitung als versprühtes Gut mit einem Dampfstrom behandelt, der - bezogen auf die Arbeitsbedingungen der Sprühzone - als überhitzter Dampf vorliegt.

2. Verfahren nach Anspruch 1, dadurch gekennzeichnet, daß man Zubereitungen entsprechender Fermenterbrühen der Behandlung mit einem Wasserdampfstrom in der Sprühzone unterwirft und dabei in der Sprühzone bevorzugt unter verringertem Druck arbeitet.

3. Verfahren nach Ansprüchen 1 und 2, dadurch gekennzeichnet, daß man die Abreicherung der Geruchsstoffe im Rahmen der Gewinnung biotechnologisch hergestellter extracellulärer Wertstoffe aus ihren wäßrigen Zubereitungen vornimmt, wobei insbesondere solche Zubereitungen zur Gewinnung von Enzymen für den Bereich der Wasch- und Reinigungsmittel aufgearbeitet werden.

4. Verfahren nach Ansprüchen 1 bis 3, dadurch gekennzeichnet, daß wäßrige Proteaselösungen desodoriert und dazu bevorzugt nach ihrer Aufkonzentration im Vakuum der Dampfbehandlung in der Sprühzone unterworfen werden.

5. Verfahren nach Ansprüchen 1 bis 4, dadurch gekennzeichnet, daß man die maximale Guttemperatur in der Sprühzone durch Vorgabe und Kontrolle des Vakuums - und damit der Siedetemperatur des Wassers unter Arbeitsbedingungen - in dieser Zone regelt.

6. Verfahren nach den Ansprüchen 1 bis 5, dadurch gekennzeichnet, daß man wäßrige Wertstoffzubereitungen desodoriert, deren Wassergehalt wenigstens 15 Gew.-%, vorzugsweise wenigstens 35 Gew.-% und insbesondere wenigstens 50 Gew.-% beträgt.

7. Verfahren nach Ansprüchen 1 bis 6, dadurch gekennzeichnet, daß man mit wäßrigen Zubereitungen temperatursensitiver Wertstoffe arbeitet und dabei die Guttemperatur in der Arbeitszone bei Werten von höchstens 45°C, vorzugsweise unterhalb von 35 bis 40°C und insbesondere bei höchstens etwa 30°C hält.

8. Verfahren nach Ansprüchen 1 bis 7, dadurch gekennzeichnet, daß man den Arbeitsdampf mit Eintrittstemperaturen im Bereich bis 250°C, vorzugsweise bis 200°C, einsetzt, wobei hier Temperaturen im Bereich von 130 bis 200°C besonders bevorzugt sein können.

9. Verfahren nach Ansprüchen 1 bis 8, dadurch gekennzeichnet, daß man die Desodorierung in der Sprühzone mit einer bevorzugt schwachen Abreicherung des Wassergehaltes im Einsatzgut verbindet.

10. Verfahren nach Ansprüchen 1 bis 9, dadurch gekennzeichnet, daß man die wäßrige Wertstoffzubereitung einer mehrfachen Behandlung in der Sprühzone unterwirft.

11. Verfahren nach Ansprüchen 1 bis 10, dadurch gekennzeichnet, daß man den Dampf im Gegenstrom zur versprühten Flüssigphase führt.

12. Anwendung des Verfahrens zur Desodorierung von fließfähigen wäßrigen Wertstoffzubereitungen durch deren Behandlung in der Sprühzone mit - bezogen auf die Arbeitsbedingungen der Sprühzone - überhitztem Wasserdampf zur Abreicherung der Geruchsstoffe aus den wäßrigen Zubereitungen, die gelöste und/oder dispergierte und auf biotechnologischem Weg gewonnene Wertstoffe enthalten.

13. Anwendung nach Anspruch 12, dadurch gekennzeichnet, daß das Verfahren auf wäßrige Kulturbrühen aus Fermentationsprozessen und insbesondere wasch- und/oder reinigungsaktive Enzyme enthaltende Fermenterlösungen angewendet wird.

14. Anwendung nach Ansprüchen 12 und 13, dadurch gekennzeichnet, daß das Verfahren auf wäßrige Proteaselösungen angewendet wird und diese vorzugsweise dabei gleichzeitig gegen unerwünschte Katalyse der Selbstzersetzung stabilisiert werden.

## Claims

1. A process for removing odorous substances from liquid aqueous preparations of useful materials from the working-up of biotechnologically obtained culture broths, characterized in that the liquid preparation is treated as a sprayed material with steam present as superheated steam under the working conditions of the spray zone.

2. A process as claimed in claim 1, characterized in that preparations of corresponding fermenter broths are subjected to the treatment with steam in the spray zone and in that the spray zone is preferably operated under reduced pressure.

3. A process as claimed in claims 1 and 2, characterized in that the odorous materials are removed during the recovery of biotechnologically produced extracellular useful materials from aqueous preparations thereof, the preparations in question being worked up in particular to recover enzymes for use in detergents.

4. A process as claimed in claims 1 to 3, characterized in that aqueous protease solutions are deodorized and, to this end, are subjected to the treatment with steam in the spray zone preferably after concentration in vacuo.

5. A process as claimed in claims 1 to 4, characterized in that the maximum material temperature in the spray zone is controlled by predetermining and monitoring the vacuum - and hence the boiling temperature under process conditions - in that zone.

6. A process as claimed in claims 1 to 5, characterized in that aqueous preparations of useful materials with a water content of at least 15% by weight, preferably at least 35% by weight and more preferably at least 50% by weight are deodorized.

7. A process as claimed in claims 1 to 6, characterized in that aqueous preparations of temperature-sensitive useful materials are treated and the material temperature in the working zone is kept at values of at most 45°C, preferably below 35 to 40°C and more preferably at most about 30°C.

8. A process as claimed in claims 1 to 7, characterized in that the entry temperatures of the steam are up to 250°C and preferably up to 200°C, temperatures in the range from 130 to 200°C being particularly preferred.

9. A process as claimed in claims 1 to 8, characterized in that deodorization in the spray zone is combined with a preferably slight reduction in the water content of the starting material.

10. A process as claimed in claims 1 to 9, characterized in that the aqueous preparation of useful materials is subjected to a multiple treatment in the spray zone.

11. A process as claimed in claims 1 to 10, characterized in that the steam flows in countercurrent to the sprayed liquid phase.

12. The use of the process for deodorizing liquid aqueous preparations of useful materials by treatment thereof with - based on the operating conditions in the spray zone - superheated steam in the spray zone to remove the odorous substances from the aqueous preparations containing dissolved and/or dispersed and biotechnologically obtained useful materials.

13. The use claimed in claim 12, characterized in that the process is applied to aqueous culture broths from fermentation processes and, in particular, fermenter solutions containing washing- and/or cleaning- active enzymes.

14. The use claimed in claims 12 and 13, characterized in that the process is applied to aqueous protease solutions which, at the same time, are stabilized against unwanted catalysis of self-decomposition processes.

## Revendications

1. Procédé d'épuisement de substances odorantes, de préparations de substances de valeur aqueuses fluides, provenant du traitement de bouillons de culture produits par voie biotechnologique,
caractérisé en ce qu'
on traite la préparation fluide sous forme de produit pulvérisé avec un courant de vapeur qui se présente, rapporté aux conditions opératoires de la zone de pulvérisation, sous forme de vapeur surchauffée.

2. Procédé selon la revendication 1,
caractérisé en ce qu'
on soumet des préparations de bouillons de fermentateur correspondants à un traitement par un courant de vapeur d'eau dans la zone de pulvérisation et pour cela on opère dans la zone de pulvérisation de préférence sous pression réduite.

3. Procédé selon les revendications 1 et 2,
caractérisé en ce qu'
on effectue l'épuisement des substances odorantes dans le cadre de la production de substances de valeur extracellulaires produites par voie biotechnologique, à partir de leurs préparations aqueuses, pour lesquelles on traite en particulier les préparations en vue de la production d'enzymes pour le domaine des produits de lavage et de nettoyage.

4. Procédé selon les revendications 1 à 3,
caractérisé en ce qu'
on désodorise des solutions aqueuses de protéase et pour cela on les soumet de préférence après leur concentration sous vide au traitement par la vapeur dans la zone de pulvérisation.

5. Procédé selon les revendications 1 à 4,
caractérisé en ce qu'
on règle la température maximale de produit dans la zone de pulvérisation par détermination et par contrôle du vide et de cette façon, de la température d'ébullition de l'eau dans les conditions opératoires, dans cette zone.

6. Procédé selon les revendications 1 à 5,
caractérisé en ce qu'
on désodorise des préparations aqueuses de substances de valeur dont la teneur en eau s'élève au moins à 15 % en poids, de préférence au moins à 35 % en poids et en particulier à au moins 50 % en poids.

7. Procédé selon les revendications 1 à 6,
caractérisé en ce qu'
on opère avec des préparations aqueuses de substances de valeur sensibles à là chaleur et pour cela on maintient la température de produit dans la zone de travail à des valeurs de, au maximum 45°C, de préférence en dessous de 35 à 40°C et en particulier à, au maximum, environ 30°C.

8. Procédé selon las revendications 1 à 7,
caractérisé en ce qu'
on net en oeuvre la vapeur de travail avec des températures d'entrée dans la plage allant jusqu'à 250°C, de préférence juncu'à 200°C, ici des températures dans la plage de 130 à 200°C pouvant être particulièrement préférées.

9. Procédé selon les revendications 1 à 8,
caractérisé en ce qu'
on relie la désodoristion dans la zone de pulvérisation à un appauvrissement de préférence faible de la teneur en eau dans le produit d'utilisation.

10. Procédé selon les revendications 1 à 9,
caractérisé en ce qu'
on soumet la préparation aqueuse de substance de valeur à un traitement multiple dans la zone de pulvérisation.

11. Procédé selon les revendications 1 à 10,
caractérisé en ce qu'
on conduit la vapeur à contre-courant à la phase liquide pulvérisée.

12. Utilisation du procédé de désodorisation de préparations aqueuses fluides de substances de valeur, par leur traitement dans la zone de pulvérisation avec, rapporté aux conditions opératoires de la zone de pulvérisation, de la vapeur d'eau surchauffée en vue de l'épuisement des substances odorantes des préparations aqueuses qui renferment des substances de valeur dissoutes et/ou dispersées et produites par voie biotechnologique.

13. Utilisation selon la revendication 12,
caractérisée en ce qu'
on utilise le procédé sur des bouillons de culture aqueux provenant des processus de fermentation et en particulier des solutions de fermentateur contenant des enzymes actifs pour le lavage et le nettoyage.

14. Utilisation selon les revendications 12 et 13,
caractérisée en ce qu'
on utilise le procédé sur des solutions aqueuses de protéases et qu'on stabilise celles-ci de préférence de cette manière en même temps contre une catalyse non désirée, de l'autodécomposition.
